# EUROPEAN PATENT SPECIFICATION

(11) **EP 2 427 766 B1**
(45) Date of publication and mention of the grant of the patent: **29.07.2015**
(21) Application number: 09844461.5
(22) Date of filing: 25.09.2009
(51) Int. Cl.: C07K 14/47, G01N 33/68

(54) **Human fg01 gene and its applications**
Menschliches fg01-Gen und ihre Anwendungen
Gène humain fg01 et ses applications

(30) Priority: 08.05.2009 US 176530 P; 19.05.2009 US 179409 P
(43) Date of publication of application: 14.03.2012
(73) Proprietor: Eli Lilly and Company, Indianapolis, IN 46285 (US)
(72) Inventor: LI, Wu-Bo, North Potomac Maryland 20878 (US); KINCH, Michael, Laytonsville Maryland 20882 (US); GOLDBLATT, Michael, McLean Virginia 22101 (US)
(74) Representative: Sexton, Jane Helen
(86) International application number: PCT/US2009/058334
(87) International publication number: WO 2010/128988

(56) References cited:
- WO-A2-2004/093804
- US-A1- 2005 196 839
- US-A1- 2009 060 987
- US-A1- 2009 118 282
- XU ET AL: "S4-01-04: Identification and characterization of a novel gene that inhibits GSK3 activity and Abeta production", ALZHEIMER'S & DEMENTIA: THE JOURNAL OF THE ALZHEIMER'SASSOCIATION, ELSEVIER, NEW YORK, NY, US, vol. 4, no. 4, 1 July 2008 (2008-07-01), pages T177-T178, XP023173428, ISSN: 1552-5260, DOI: 10.1016/J.JALZ.2008.05.475 [retrieved on 2008-07-01]
- DATABASE EMBL [Online] 8 November 2000 (2000-11-08), "Homo sapiens genomic DNA, chromosome 8q23, clone: KB1589B1.", XP002683693, retrieved from EBI accession no. EM_HU:AP002906 Database accession no. AP002906
- CARNINCI P ET AL: "NORMALIZATION AND SUBTRACTION OF CAP-TRAPPER-SELECTED CDNAS TO PREPARE FULL-LENGTH CDNA LIBRARIES FOR RAPID DISCOVERY OF NEW GENES", GENOME RESEARCH, COLD SPRING HARBOR LABORATORY PRESS, WOODBURY, NY, US, vol. 10, no. 10, 1 January 2000 (2000-01-01), pages 1617-1630, XP002944079, ISSN: 1088-9051, DOI: 10.1101/GR.145100 & DATABASE UniProt [Online] 1 March 2003 (2003-03-01), "SubName: Full=Uncharacterized protein;", XP002683694, retrieved from EBI accession no. UNIPROT:Q8C7T0 Database accession no. Q8C7T0
- YUN-WU ZHANG ET AL: "A Functional Mouse Retroposed Gene Rps23r1 Reduces Alzheimer's [beta]-Amyloid Levels and Tau Phosphorylation", NEURON, vol. 64, no. 3, 1 November 2009 (2009-11-01), pages 328-340, XP55038416, ISSN: 0896-6273, DOI: 10.1016/j.neuron.2009.08.036 & YUN-WU ZHANG ET AL: "Supplemental DATA to : A Functional Mouse Retroposed to : Gene Rps23r1 Reduces Alzheimer's [beta]-Amyloid Levels and Tau Phosphorylation", NEURON, vol. 64, no. 3, 1 November 2009 (2009-11-01), pages 1-16, XP002683695, ISSN: 0896-6273, DOI: 10.1016/j.neuron.2009.08.036
- XU: 'S4-01-04: Identification and characterization of a novel gene that inhibits GSK3 activity and Abeta production.' ALZHEIMER'S AND DEMENTIA vol. 4, no. 4, 2008, pages T177 - T178
- ZHANG ET AL.: 'A functional mouse retroposed gene Rps23r1 reduces Alzheimer's beta-amyloid levels and tau phosphorylation.' NEURON. vol. 64, no. 3, 12 November 2009, pages 328 - 340

## Description

### BACKGROUND OF THE INVENTION

### Field of the Invention

This invention involves the identification of a human gene implicated in the development of Alzheimer's Disease (AD) and methods of enhancing expression of that gene or the protein it encodes to inhibit, treat, reduce and possibly reverse symptoms associated with AD.

### Related Art

This application is related to the discovery set forth in U.S. Patent Application Serial No. 12/399,850 (published as US2009/0233993) of a mouse gene *fg0l* that expresses a mouse protein fg01 which appears to inhibit activity of the serine/threonine kinase GSK3. Inhibition or down-regulation of this enzyme has been shown to reduce certain symptoms associated with AD, including the accumulation of Aβ, a cleavage product of APP which is found in plaques and other physiognomy associated with AD. Named inventors herein are also inventors named in 12/399,850. That application identifies a mouse gene, and the protein it encodes, that were identified by a process called Random Homologous Gene Perturbation (RHGP), which permits random insertion of a gene search vector which, when inserted in the allele of a eukaryotic gene, generates an antisense or sense RNA sequence, which inactivates or activates the matching allele. This process allows inspection of the entire eukaryotic genome of a cell, to identify specific targets for manipulation. It is disclosed in U.S. Patent Application Serial No. 11/928,393 (published as US2009/0136927). In this application, the human gene, a prerequisite for fashioning therapeutic treatment based on that gene and its encoded protein, are set forth. Humanfg0l and the encoded human fg0l protein offer opportunities to screen for those patients who can be effectively treated to control the development of AD symptoms, as well as opportunities for therapeutic intervention.

### Background of the Technology

AD is a progressive and generally fatal neural disease. Symptoms reflected by AD patients include profound memory loss, a reduction in higher order thinking and aberrant behavior. Currently, there is no known cure, although a number of treatments for the symptoms described are being explored. In part due to its progressive nature, the toll taken by AD patients is enormous, on their resources, and those of care-givers.

There are two profound brain structures that are associated with the "AD phenotype". These are generally referred to as "plaques" and "tangles". Plaques reflect the excessive production and accumulation of the β-amyloid peptide (Aβ) that is the product of cleavage of the protein APP. Genetic and chemical studies have shown that a variety of pathogenic mutations in the APP gene and in genes encoding proteins known as presenilins 1 and 2 (PS1 and PS2), the major component of the gamnia-Secretase complex, increase the production of Aβ peptide. A mouse model of AD, where the mice exhibit early onset of plaque formation, and hyperphosphorylation of another protein, tau, which is typically found within dead or dying neuronal cells in the form of tangles, which are comprised largely of tau proteins so phosphorylated that they have been rendered insoluble, and appear in the form of filaments, is disclosed in U.S. Patent 5,898,094. Related research gave rise to a mouse model with a human tau transgene, to better model AD, as set forth in U.S. Patent 7,161,060.

Subsequently, in U.S. Patent Application Serial No. 12/399,850 (published as US2009/0233993), the identification of a gene in mice having a direct correlation with the "AD phenotype" is reported, by the current inventors and others. Although upregulation of the gene and protein encoded thereby, mouse *fg*0l and mouse fg0l, appeared to offer some inhibition of the cleavage patterns that give rise to the AD phenotype, suppressing plaque and tangle formation, extensive research indicated that no human analogue existed. (*Neuron,* Zhang et al, in press). While transgenic modification of human cells to express the mouse *fg*01 gene indicated the value of upregulation, the protein encoded by this mouse gene is immunogenic, and does not offer a method for treatment of humans.

Xu et al, S4-01-04, Alzheimer's & Dementia: The Journal of The Alzheimer's Association, Elsevier, New York, NY, US, vol.4, no.4, 1 July 2008 refers to the identification and characterization of a novel gene that inhibits GSK3 activity and Abeta production. EBI accession no. EM_HU:AP002906 dated 8 November 2000 refers to Homo sapiens genomic DNA, chromosome 8q23, clone KB1589B1. WO 2004/093804 A2 refers to human polypeptides encoded by polynucleotides and methods of their use. Carninci P et al, Genome Research, Cold Spring Harbor Laboratory Press, Woodbury, NY, US, vol. 10, no. 10, 1 January 2000 refers to normalization and subtraction of cap-trapper-selected cDNAs to prepare full-length cDNA libraries for rapid discovery of new genes. US 2005/196839 A1 refers to beta-secretase enzyme compositions and methods of use thereof.

### SUMMARY OF THE INVENTION

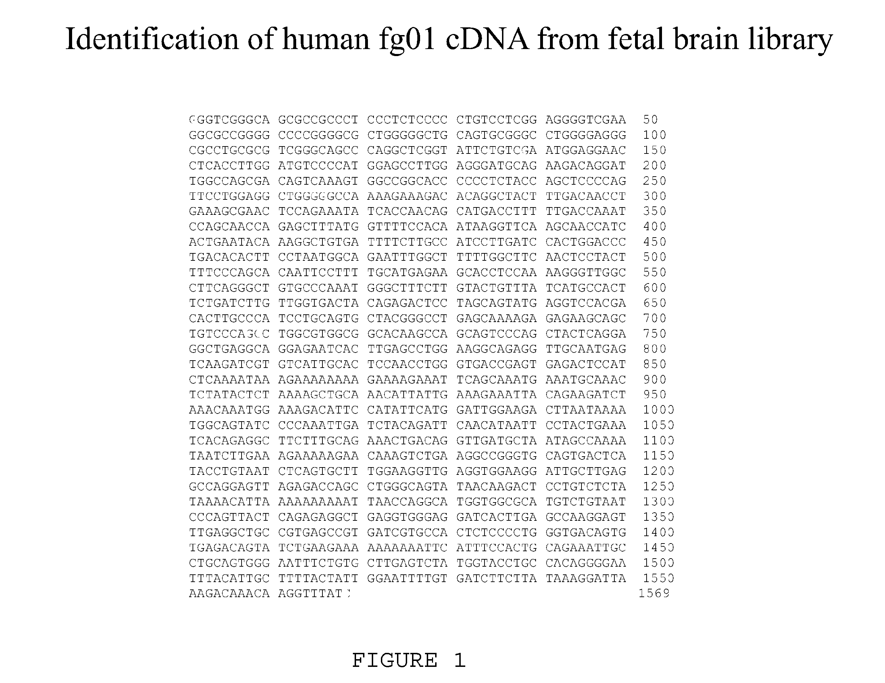

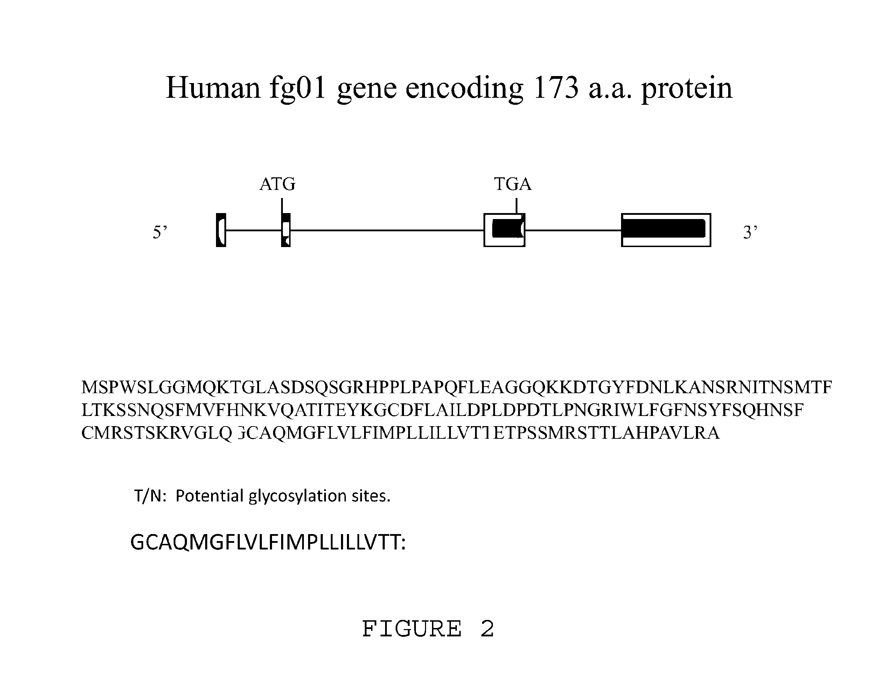

The human sequence for *fg*0l is set forth in Figure 1, encoding a 173 amino acid protein, human fg0l, set forth at Figure 2. The protein is a type 1b transmembrane proteins, similar in general structure to the mouse fg0l. It shares less than 70% homology with the mouse analogue, however, which is the basis for the immunogenicity of the mouse fg01 protein.

Mouse FG01 was identified in an RHGP-based campaign to identify novel regulators of Aβ production. The regulation of Aβ is understood to be a key marker of Aizheimer's Disease (AD) damage and inhibitors of Aβ could provide much-needed opportunities to prevent or treat this disease.

The RHGP campaign utilized a murine cell line and hypothesized a mechanism in which fg0l, a transmembrane protein, induces the enzymatic activity of adenylyl cyclase, which promotes the production of cAMP and in turn activates protein kinase A (PKA). PKA activation then serves to inhibit GSK3 kinase activity and thereby prevents the phosphorylation of tau. These activities serve to decrease Aβ production and thus decrease or prevent the deposition of amyloid plaques, the hallmark of the manifestation of Alzheimer's disease. Altogether, these results suggest that upregulation of human fg0l could be used to decrease the cellular pathogenicity of Alzheimer's disease.

Based on these findings, therapeutics that directly upregulate human *fg*01 expression (e.g., via gene therapy), indirectly upregulate *fg* 01 expression (e.g., inducers of endogenous human *fg* 01 expression or that mimic *fg* 01 expression (e.g., that activate adenylyl cyclase, increase cAMP, inhibit GSK3 or prevent phosphorylation of tau) could similarly have utility for the management of Alzheimer's Disease.

Accordingly, the invention provides an isolated nucleic acid that encodes a protein having the amino acid sequence

MSPWSLGGMQKTGLASDSQSGRHPPLPAPQFLEAGGQKKDTGYFDNLKANSRNI
TNSMTFLTKSSNQSFMVFHNKVQATITEYKGCDFLAILDPLDPDTLPNGRIWLFGF
NSYFSQHNSFCMRSTSKRVGLQGCAQMGFLVLFIMPLLILLVTTETPSSMRSTTLA
HPAVLRA.

The invention also provides an isolated protein having the amino acid sequence encoded by the nucleic acid of Figure 1.

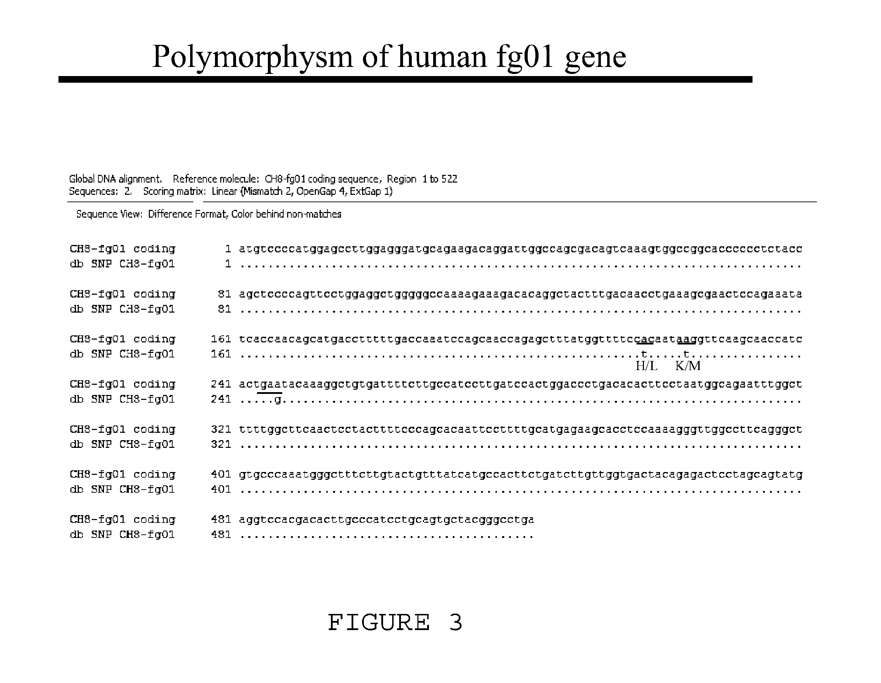

Furthermore, the invention provides an *in vitro* or *ex vivo* method of suppressing the formation of plaques of amyloid-β in host cells, comprising increasing the level of fg01 protein in said cells by gene therapy, wherein the fg01 protein has an amino acid sequence encoded by the nucleic acid of Figure 1 or encoded by the nucleic acid of Figure 1 which is altered by substitution of one or more nucleotides corresponding to any one of the single nucleotide polymorphisms shown in Figure 3, said single nucleotide polymorphisms being adenine substituted with thymine at position 218, adenine substituted with thymine at position 224 and adenine substituted with guanine at position 246.

The invention also provides fg01 protein for use in a method of suppressing the formation of plaques of amyloid-β in host cells *in vivo,* wherein said method comprises increasing the level of the fg01 in said cells by gene therapy and wherein the fg01 protein has an amino acid sequence encoded by the nucleic acid of Figure 1 or encoded by the nucleic acid of Figure 1 which is altered by substitution of one or more nucleotides corresponding to any one of the single nucleotide polymorphisms shown in Figure 3, said single nucleotide polymorphisms being adenine substituted with thymine at position 218, adenine substituted with thymine at position 224 and adenine substituted with guanine at position 246.

The invention also provides an isolated nucleic acid having the sequence of Figure 1, wherein said sequence is altered by substitution of one or more nucleotides corresponding to any one of the single nucleotide polymorphisms shown in Figure 3, said single nucleotide polymorphisms being adenine substituted with thymine at position 218, adenine substituted with thymine at position 224 and adenine substituted with guanine at position 246.

### BRIEF DESCRIPTION OF THE DRAWINGS

The accompanying drawings, which are incorporated herein and constitute part of this specification, illustrate exemplary embodiments of the invention, and, together with the general description given above and the detailed description given below, serve to explain the features of the invention.
**Figure 1****: Human *fg* 01 cDNA sequence.** Provides the sequence of human *fg* 01 cDNA obtained from a fetal brain tissue library. The start ATG and stop codon TGA are in red. The intron and exon boundaries are shown in green.
**Figure 2****: Human fg0l protein sequence.** The human fg01 protein sequence encoded by the fg01 gene is given in Figure 2, from N- to C-terminus. The transmembrane domain of human fg01 protein is given in blue, and several potential glycosylation sites are set forth in red.
**Figure 3****: Human *fg*01 polymorphism.** Certain sites in the human *fg*01gene are identified as polymorphic, with identified mutations indicated.

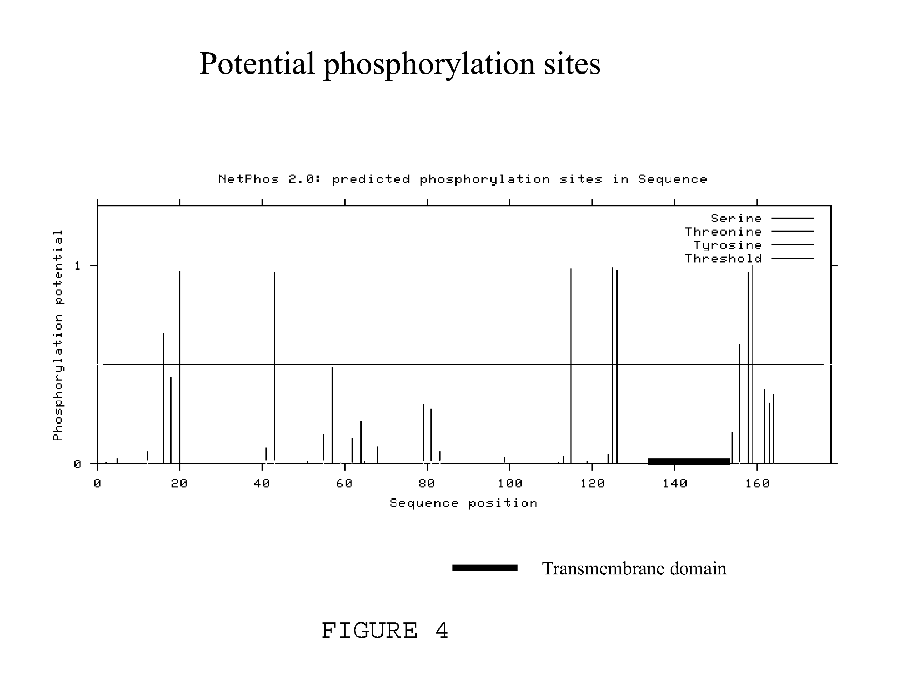

**Figure 4****: Potential Phosphorylation Sites.** The human fg01 protein is likely activated by phosphorylation. It exhibits a number of phosphorylation sites both intracellularly and beyond the transmembrane domain, in the extracellular portion of the molecule. These potential phosphorylation sites are identified in Figure 4.

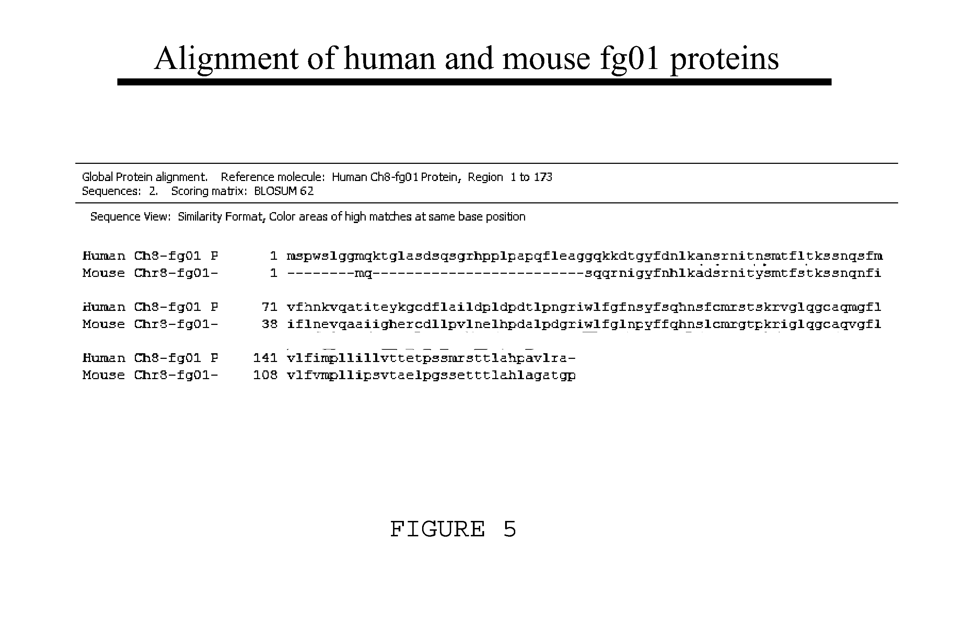

**Figure 5****: Alignment with murine fg01.** The search for the human *fg*01 gene was inspired by identification of the murine *fg*01 gene, described in U.S. Patent Application Serial Number 12/399,850 (published as US2009/0233993) and in *Neuron,* Zhang et al (Neuron 64, pages 328-340, November 12, 2009). Although those researchers found no human counterpart, an alignment of the human and murine fg0l proteins is set forth in Figure 5.

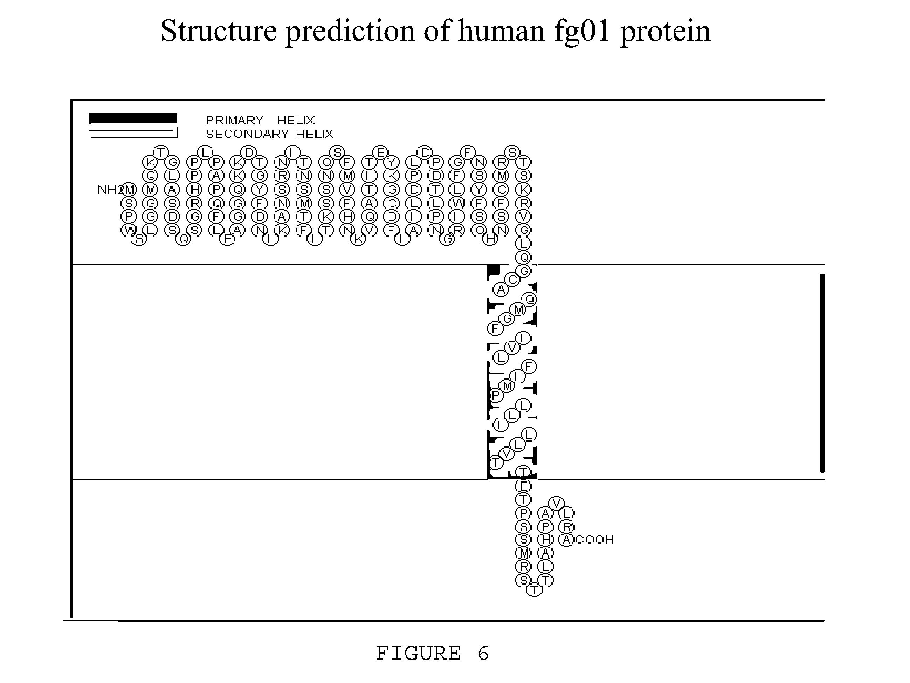

**Figure 6****: Structure prediction for human fg0l. Based on the sequence of human** protein fg01, and the conformance of similar proteins, the conformational structure of human fg0l, with amino acid residue identification inserted, is set forth in Figure 6.

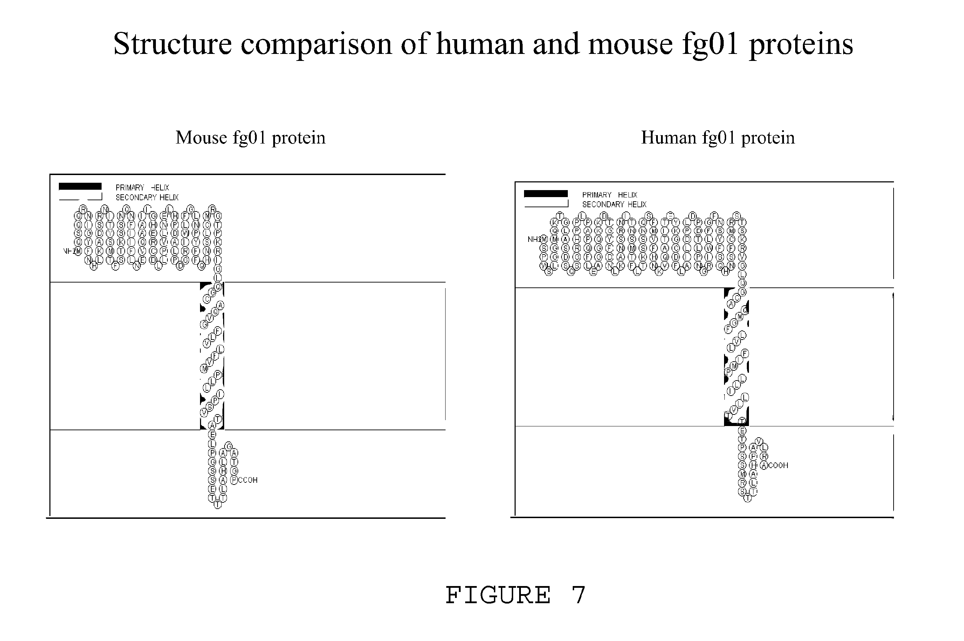

**Figure 7****: Structure comparison of human and murine fg01 proteins. The** nominal three dimensional structure of the human and mouse fg01 proteins are compared in Figure 7, showing a conserved structure although the sequence homology is less than 70%.

### DESCRIPTION OF THE INVENTION

To identify the human fg01 homolog, the human genome browser search indicated that a genomic DNA domain in human chromosome 8 shares relatively high homology with the mouse fg01 coding sequence. Although there is no human mRNA and EST sequence information available in that locus in GeneBank, the 5' and 3' cDNA sequences were amplified by PCR from a human fetal brain cDNA library constructed in a cloning vector using the human chromosome 8-specific primers along with the primers designed from cDNA cloning vector. A 1569-bop full-length cDNA sequence was reconstituted from the PCR products. The cDNA sequence was also confirmed by a separate RT-PCR from a total RNA of human fetal brain.

The cDNA sequence perfectly matches human chromosome 8 and contains 4 exons spanning a 6.7 kb genomic contiq. The first exon is located within the CpG island region. An open reading frame encoding a 173 amino acid protein is located within exon 2 and 3. Similar to the mouse fg01 protein, human fg01 homolog is a type 1b transmembrane protein. Human and mouse fg01 proteins share a degree of homology somewhat less than 70%.

Three potential glycosylation sites are identified in the extracellular domain of the human fg01 protein by a computer prediction program. In addition, the computer prediction program also identified several potential phosphorylation sites in both intracellular and extracellular domains of the protein. Similar transmembrane proteins are activated by phosphorylation intracellularly, causing a change in conformation and sometimes activity.

The database search revealed that three SNPs (Single-Nucleotide Polymorphisms) are involved in the coding region leading to 2 amino acids changed. These amino acid changes may be related to the AD pathogenesis, and in particular, presentation of the AD phenotype.

Enhancing expression of the *fg*01 gene is indicated to be effective in suppressing development of the AD phenotype. In particular, enhanced expression (over expression) of the *fg*01 gene delays and reduces the formation of plaques commonly associated with AD. The presence of fg01 protein may well suppress the abnormal cleavage of APP leading to accumulation of Aβ and phosphorylation of tau. This offers several different embodiments for intervention to either delay or prevent AD onset, or treat AD to prevent the symptoms from progressing. Methods of enhancing expression of a gene through targeted gene therapy are well known.

In a first alternative, human fg0l could function identically to murine fg0l as described in U.S. Patent Application Serial Number 12/399,850. This could arise if fg0l interacts with the cell membrane as a peripheral membrane protein or if it interacts with the cell membrane indirectly via other proteins (e.g., *cis*-interactions with membrane spanning proteins or via post-translational modifications (e.g., myristoylation) that facilitate membrane interactions. In this scenario, the strategies generally employed to enhance gene expression, through gene therapy, may be used. Thus, the cell genome may be transformed to include multiple copies of the gene, either by transfection with a plasmid incorporating the human *fg*0l gene operatively linked to a regulatory sequence which enables its expression (e.g., a promoter) or inserted downstream of an active promoter. The cell may be modified to include an amplifiable gene such as DHFR, and exposed to stress such as a toxin like methotrexate to induce amplification of the amplifiable gene and those in its vicinity, which would include the *fg*0l of the native genome, the DHFR gene having been placed in proximity to the *fg*01 gene.

Alternatively, gene expression may be upregulated by insertion of promoter and/or enhancer elements up-or-downstream of the genomic transcript, enhancing expression of the gene. These and other methods of enhancing expression through alteration of the human genome, either by insertion of copies of the gene, or alteration of the genome to enhance expression of the gene, are set forth in U.S. Patent 5,272,071.

Gene therapy to enhance the expression levels of fg0l protein may be effected in vivo, by introducing transfection plasmids into the host organism cells. Alternatively, they may be effected *ex vivo,* wherein host cells are transformed *in vitro* and then introduced back into the host. And of course, they may be effected *in vitro.*

In addition, if human fg01 protein interacts with the outer cell membrane as a peripheral membrane or soluble (not directly attached to the membrane but via interactions with other proteins, then ectopic delivery of fg0l protein might be sufficient to mediate the same types of effects observed via overexpression of membrane-associated (murine) fg0l. In this case, treatment of patients with wild type or preferably recombinant human fg01 could have utility for treating Alzheimer's disease or other indications associated with deposition of Aβ. Likewise, derivatives of human fg01 (e.g., fusion proteins) could serve the same purpose.

Human fg01 may mediate its effects via *trans* interactions to other membrane associated proteins (much like a soluble growth factor stimulates its receptor) and that this activity might be mimicked using other means to stimulate its ligands. In this case, small molecule (chemical entities, aptamers) or biologies (e.g., antibodies, avimers) that stimulate the same receptor or signaling system could have utility for the treatment of Alzheimer's Disease. In particular, the generation of suitable antibodies using either conventional host immunogenic generation as taught by Kohlerr-Milstein, followed by humanization of the CDRs, or phage display, to provide human antibodies, may be utilized. Antibodies effective therapeutically with other transmembrane proteins, such as the antibodies in Herceptin® and Avastin® are known to those of skill in the art.

Fundamentally, the identification of the human *fg*0l gene and the fg0l protein encoded thereby opens the door for enhanced treatment of Alzheimer's Disease, and enhanced diagnostics. Current diagnostics are largely based on partially subjective testing - degree of loss of cognitive function, higher order reasoning and the like. Although the presence of a large or pronounced amount of plaques and tangles, i.e., the presentation of the AD phenotype, may be indicative of progressive AD, identification of the norm for any particular subject, or the baseline for a population, remains elusive.

By screening for the expression of human *fg*0l, as well as the presence of the human protein *fg*0l, one can identify those patient's at a potentially higher risk of developing AD. Patients presenting with developing symptoms may be screened for level of *fg*0l in the brain and body, which may allow rapid identification of those whose progression toward profound AD whom might be better or more immediately treated with therapeutics that delay the onset of AD symptoms, like Aricept® (donepezil hydrochloride). The same screening may allow the identification of preferred routes of treatment, either through gene therapy, or through administration of *fg*0l protein, or a small molecule or biologic which enhances the action of *fg*0l, depending on, for example, the frequency of *fg*0l transcripts, mRNA concentrations and circulating protein levels.

The invention disclosed herein resides in the identification of novel gene sequences and protein sequences. These genes and proteins occur naturally, and thus the invention herein resides in their identification as isolated and detectable forms. By isolated, the inventors herein intend that the indicated nucleic acid sequence, or amino acid sequence, has been separated from the chromosome on which it is found (Chromosome 8) or from the cytoplasm and cell and cell debris found in the extracellular matrix, such that the nucleic acid or protein can be identified and manipulated. Purification to a therapeutic level is contemplated, but not a requirement for this invention, or its use.

This invention has been described in terms of the nucleic acid sequence for the identified gene, and the amino acid sequence of the corresponding protein. Those of skill in the art are well aware of alterations to the nucleotide sequence that may be introduced without affecting the protein expressed, including truncation methods, and base alterations that lead to enhanced expression through selection of preferred codons. By the same token, amino acid substitutions that do not disturb the hydropathic index of the human fg0l protein, or truncate those portions of the molecule not involved in binding or structure determination, are familiar to practitioners in this art.

## Claims

1. An isolated nucleic acid that encodes a protein having the amino acid sequence:

MSPWSLGGMQKTGLASDSQSGRHPPLPAPQFLEAGGQKKDTGYFDNLKANSR
NITNSMTFLTKSSNQSFMVFHNKVQATITEYKGCDFLAILDPLDPDTLPNGRIWL
FGFNSYFSQHNSFCMRSTSKRVGLQGCAQMGFLVLFIMPLLILLVTTETPSSMRS
TTLAHPAVLRA.

2. The nucleic acid of Claim 1, which comprises the sequence of Figure 1.

3. The nucleic acid of Claim 2, wherein said nucleic acid consists of the sequence of Figure 1.

4. The nucleic acid of Claim 1, wherein said nucleic acid has a sequence which varies from the sequence of Figure 1 by nucleotide bases which do not alter a protein encoded thereby.

5. An isolated protein having the amino acid sequence encoded by the nucleic acid of Figure 1.

6. The protein of Claim 5, wherein said protein comprises the amino acid sequence:

MSPWSLGGMQKTGLASDSQSGRHPPLPAPQFLEAGGQKKDTGYFDNLKANSR
NITNSMTFLTKSSNQSFMVFHNKVQATITEYKGCDFLAILDPLDPDTLPNGRIWL
FGFNSYFSQHNSFCMRSTSKRVGLQGCAQMGFLVLFIMPLLILLVTTETPSSMRS
TTLAHPAVLRA.

7. The protein of Claim 6, wherein said protein consists of the amino acid sequence:

MSPWSLGGMQKTGLASDSQSGRHPPLPAPQFLEAGGQKKDTGYFDNLKANSR
NITNSMTFLTKSSNQSFMVFHNKVQATITEYKGCDFLAILDPLDPDTLPNGRIWL
FGFNSYFSQHNSFCMRSTSKRVGLQGCAQMGFLVLFIMPLLILLVTTETPSSMRS
TTLAHPAVLRA.

8. An *in vitro* or *ex vivo* method of suppressing the formation of plaques of amyloid-β in host cells, comprising increasing the level of fg01 protein in said cells by gene therapy, wherein the fg01 protein has an amino acid sequence encoded by the nucleic acid of Figure 1 or encoded by the nucleic acid of Figure 1 which is altered by substitution of one or more nucleotides corresponding to any one of the single nucleotide polymorphisms shown in Figure 3, said single nucleotide polymorphisms being adenine substituted with thymine at position 218, adenine substituted with thymine at position 224 and adenine substituted with guanine at position 246.

9. Fg01 protein for use in a method of suppressing the formation of plaques of amyloid-β in host cells *in vivo,* wherein said method comprises increasing the level of the fg01 in said cells by gene therapy and wherein the fg01 protein has an amino acid sequence encoded by the nucleic acid of Figure 1 or encoded by the nucleic acid of Figure 1 which is altered by substitution of one or more nucleotides corresponding to any one of the single nucleotide polymorphisms shown in Figure 3, said single nucleotide polymorphisms being adenine substituted with thymine at position 218, adenine substituted with thymine at position 224 and adenine substituted with guanine at position 246.

10. The method of claim 8 or the fg01 protein for use in the method of claim 9, wherein the fg01 protein comprises the amino acid sequence:

MSPWSLGGMQKTGLASDSQSGRHPPLPAPQFLEAGGQKKDTGYFDNLKANSR
NITNSMTFLTKSSNQSFMVFHNKVQATITEYKGCDFLAILDPLDPDTLPNGRIWL
FGFNSYFSQHNSFCMRSTSKRVGLQGCAQMGFLVLFIMPLLILLVTTETPSSMRS
TTLAHPAVLRA.

11. The method of claim 8 or the fg01 protein for use in the method of claim 9, wherein the fg01 protein consists of the amino acid sequence:

MSPWSLGGMQKTGLASDSQSGRHPPLPAPQFLEAGGQKKDTGYFDNLKANSR
NITNSMTFLTKSSNQSFMVFHNKVQATITEYKGCDFLAILDPLDPDTLPNGRIWL
FGFNSYFSQHNSFCMRSTSKRVGLQGCAQMGFLVLFIMPLLILLVTTETPSSMRS
TTLAHPAVLRA.

12. The method of claim 8 or the fg01 protein for use in the method of claim 9, wherein the host cells are human brain cells.

13. An isolated nucleic acid having the sequence of Figure 1, wherein said sequence is altered by substitution of one or more nucleotides corresponding to any one of the single nucleotide polymorphisms shown in Figure 3, said single nucleotide polymorphisms being adenine substituted with thymine at position 218, adenine substituted with thymine at position 224 and adenine substituted with guanine at position 246.

## Patentansprüche

1. Isolierte Nucleinsäure, die ein Protein codiert, das die Aminosäuresequenz: 
MSPWSLGGMQKTGLASDSQSGRHPPLPAPQFLEAGGQKKDTGYFDNLKANSR
NITNSMTFLTKSSNQSFMVFHNKVQATITEYKGCDFLAILDPLDPDTLPNGRIWL
FGFNSYFSQHNSFCMRSTSKRVGLQGCAQMGFLVLFIMPLLILLVTTETPSSMRS
TTLAHPAVLRA
 hat.

2. Nucleinsäure gemäß Anspruch 1, die die Sequenz von Figur 1 umfasst.

3. Nucleinsäure gemäß Anspruch 2, wobei die Nucleinsäure aus der Sequenz von Figur 1 besteht.

4. Nucleinsäure gemäß Anspruch 1, wobei die Nucleinsäure eine Sequenz hat, die sich von der Sequenz von Figur 1 durch Nucleotidbasen unterscheidet, die ein dadurch codiertes Protein nicht verändern.

5. Isoliertes Protein, das die Aminosäuresequenz hat, die durch die Nucleinsäure von Figur 1 codiert wird.

6. Protein gemäß Anspruch 5, wobei das Protein die Aminosäuresequenz: 
MSPWSLGGMQKTGLASDSQSGRHPPLPAPQFLEAGGQKKDTGYFDNLKANSR
NITNSMTFLTKSSNQSFMVFHNKVQATITEYKGCDFLAILDPLDPDTLPNGRIWL
FGFNSYFSQHNSFCMRSTSKRVGLQGCAQMGFLVLFIMPLLILLVTTETPSSMRS
TTLAHPAVLRA
 umfasst.

7. Protein gemäß Anspruch 6, wobei das Protein aus der Aminosäuresequenz: 
MSPWSLGGMQKTGLASDSQSGRHPPLPAPQFLEAGGQKKDTGYFDNLKANSR
NITNSMTFLTKSSNQSFMVFHNKVQATITEYKGCDFLAILDPLDPDTLPNGRIWL
FGFNSYFSQHNSFCMRSTSKRVGLQGCAQMGFLVLFIMPLLILLVTTETPSSMRS
TTLAHPAVLRA
 besteht.

8. In-vitro- oder Ex-vivo-Verfahren zur Unterdrückung der Bildung von Amyloid-β-Plaques in Wirtszellen, das Erhöhen des Levels von fg01-Protein in den Zellen durch Gentherapie umfasst, wobei das fg01-Protein eine Aminosäuresequenz hat, die durch die Nucleinsäure von Figur 1 codiert wird oder durch die Nucleinsäure von Figur 1, die durch Substitution eines oder mehrerer Nucleotide, entsprechend einem der in Figur 3 gezeigten Einzelnucleotidpolymorphismen, verändert ist, codiert wird, wobei die Einzelnucleotidpolymorphismen Adenin, substituiert durch Thymin in Position 218, Adenin, substituiert durch Thymin in Position 224 und Adenin, substituiert durch Guanin in Position 246 sind.

9. Fg01-Protein zur Verwendung in einem Verfahren zur Unterdrückung der Bildung von Amyloid-β-Plaques in Wirtszellen in vivo, wobei das Verfahren Erhöhen des Levels von fg01 in den Zellen durch Gentherapie umfasst und wobei das fg01-Protein eine Aminosäuresequenz hat, die durch die Nucleinsäure von Figur 1 codiert wird, oder durch die Nucleinsäure von Figur 1, die durch Substitution eines oder mehrerer Nucleotide, entsprechend einem der in Figur 3 gezeigten Einzelnucleotidpolymorphismen, verändert ist, codiert wird, wobei die Einzelnucleotidpolymorphismen Adenin substituiert durch Thymin in Position 218, Adenin substituiert durch Thymin in Position 224 und Adenin substituiert durch Guanin in Position 246 sind.

10. Verfahren gemäß Anspruch 8 oder fg01-Protein zur Verwendung in dem Verfahren gemäß Anspruch 9, wobei das fg01-Protein die Aminosäuresequenz: 
MSPWSLGGMQKTGLASDSQSGRHPPLPAPQFLEAGGQKKDTGYFDNLKANSR
NITNSMTFLTKSSNQSFMVFHNKVQATITEYKGCDFLAILDPLDPDTLPNGRIWL
FGFNSYFSQHNSFCMRSTSKRVGLQGCAQMGFLVLFIMPLLILLVTTETPSSMRS
TTLAHPAVLRA
 umfasst.

11. Verfahren gemäß Anspruch 8 oder fg01-Protein zur Verwendung in dem Verfahren gemäß Anspruch 9, wobei das fg01-Protein aus der Aminosäuresequenz: 
MSPWSLGGMQKTGLASDSQSGRHPPLPAPQFLEAGGQKKDTGYFDNLKANSR
NITNSMTFLTKSSNQSFMVFHNKVQATITEYKGCDFLAILDPLDPDTLPNGRIWL
FGFNSYFSQHNSFCMRSTSKRVGLQGCAQMGFLVLFIMPLLILLVTTETPSSMRS
TTLAHPAVLRA
 besteht.

12. Verfahren gemäß Anspruch 8 oder fg01-Protein zur Verwendung in dem Verfahren gemäß Anspruch 9, wobei die Wirtszellen humane Gehirnzellen sind.

13. Isolierte Nucleinsäure, die die Sequenz von Figur 1 hat, wobei die Sequenz durch Substitution eines oder mehrerer Nucleotide, entsprechend einem der in Figur 3 gezeigten Einzelnucleotidpolymorphismen, verändert ist, wobei die Einzelnucleotidpolymorphismen Adenin substituiert durch Thymin in Position 218, Adenin substituiert durch Thymin in Position 224 und Adenin substituiert durch Guanin in Position 246 sind.

## Revendications

1. Acide nucléique isolé qui code une protéine comportant la séquence d'acides aminés suivante :

MSPWSLGGMQKTGLASDSQSGRHPPLPAPQFLEAGGQKKDTGYFDNLKANSR
NITNSMTFLTKSSNQSFMVFHNKVQATITEYKGCDFLAILDPLDPDTLPNGRIWL
FGFNSYFSQHNSFCMRSTSKRVGLQGCAQMGFLVLFIMPLLILLVTTETPSSMRS
TTLAHPAVLRA.

2. Acide nucléique conforme à la revendication 1, qui comprend la séquence indiquée sur la Figure 1.

3. Acide nucléique conforme à la revendication 2, lequel acide nucléique consiste en la séquence indiquée sur la Figure 1.

4. Acide nucléique conforme à la revendication 1, lequel acide nucléique possède une séquence qui diffère de la séquence indiquée sur la Figure 1 par des bases de nucléotides qui ne modifient pas la protéine codée par cette séquence-ci.

5. Protéine isolée présentant la séquence d'acides aminés codée par l'acide nucléique représenté sur la Figure 1.

6. Protéine conforme à la revendication 5, laquelle protéine comporte la séquence d'acides aminés suivante :

MSPWSLGGMQKTGLASDSQSGRHPPLPAPQFLEAGGQKKDTGYFDNLKANSR
NITNSMTFLTKSSNQSFMVFHNKVQATITEYKGCDFLAILDPLDPDTLPNGRIWL
FGFNSYFSQHNSFCMRSTSKRVGLQGCAQMGFLVLFIMPLLILLVTTETPSSMRS
TTLAHPAVLRA.

7. Protéine conforme à la revendication 6, laquelle protéine consiste en la séquence d'acides aminés suivante :

MSPWSLGGMQKTGLASDSQSGRHPPLPAPQFLEAGGQKKDTGYFDNLKANSR
NITNSMTFLTKSSNQSFMVFHNKVQATITEYKGCDFLAILDPLDPDTLPNGRIWL
FGFNSYFSQHNSFCMRSTSKRVGLQGCAQMGFLVLFIMPLLILLVTTETPSSMRS
TTLAHPAVLRA.

8. Procédé *in vitro* ou *ex vivo* de suppression de la formation de plaques de protéine β-amyloïde chez des cellules hôtes, lequel procédé comporte le fait de faire augmenter par thérapie génique le niveau de protéine fg01 dans lesdites cellules, étant entendu que la protéine fg01 présente une séquence d'acides aminés qui est codée soit par l'acide nucléique représenté sur la Figure 1, soit par un acide nucléique dérivé de celui représenté sur la Figure 1 par remplacement d'un ou de plusieurs nucléotide(s) correspondant à l'un des polymorphismes mononucléotidiques indiqués sur la figure 3, lesquels polymorphismes mononucléotidiques sont le remplacement de l'adénine en position 218 par une thymine, le remplacement de l'adénine en position 224 par une thymine, et le remplacement de l'adénine en position 246 par une guanine.

9. Protéine fg01 pour utilisation dans un procédé de suppression *in vivo* de la formation de plaques de protéine β-amyloïde chez des cellules hôtes, lequel procédé comporte le fait de faire augmenter par thérapie génique le niveau de protéine fg01 dans lesdites cellules, et laquelle protéine fg01 présente une séquence d'acides aminés qui est codée soit par l'acide nucléique représenté sur la Figure 1, soit par un acide nucléique dérivé de celui représenté sur la Figure 1 par remplacement d'un ou de plusieurs nucléotide(s) correspondant à l'un des polymorphismes mononucléotidiques indiqués sur la figure 3, lesquels polymorphismes mononucléotidiques sont le remplacement de l'adénine en position 218 par une thymine, le remplacement de l'adénine en position 224 par une thymine, et le remplacement de l'adénine en position 246 par une guanine.

10. Procédé conforme à la revendication 8, dans lequel la protéine fg01, ou protéine fg01 pour utilisation dans le procédé indiqué dans la revendication 9, laquelle protéine fg01, comporte la séquence d'acides aminés suivante :

MSPWSLGGMQKTGLASDSQSGRHPPLPAPQFLEAGGQKKDTGYFDNLKANSR
NITNSMTFLTKSSNQSFMVFHNKVQATITEYKGCDFLAILDPLDPDTLPNGRIWL
FGFNSYFSQHNSFCMRSTSKRVGLQGCAQMGFLVLFIMPLLILLVTTETPSSMRS
TTLAHPAVLRA.

11. Procédé conforme à la revendication 8, dans lequel la protéine fg01, ou protéine fg01 pour utilisation dans le procédé indiqué dans la revendication 9, laquelle protéine fg01, consiste en la séquence d'acides aminés suivante :

MSPWSLGGMQKTGLASDSQSGRHPPLPAPQFLEAGGQKKDTGYFDNLKANSR
NITNSMTFLTKSSNQSFMVFHNKVQATITEYKGCDFLAILDPLDPDTLPNGRIWL
FGFNSYFSQHNSFCMRSTSKRVGLQGCAQMGFLVLFIMPLLILLVTTETPSSMRS
TTLAHPAVLRA.

12. Procédé conforme à la revendication 8, ou protéine fg01 pour utilisation dans le procédé indiqué dans la revendication 9, dans lequel procédé les cellules hôtes sont des cellules de cerveau humain.

13. Acide nucléique isolé présentant la séquence représentée sur la Figure 1, dans lequel ladite séquence est modifiée par remplacement d'un ou de plusieurs nucléotide(s) correspondant à l'un des polymorphismes mononucléotidiques indiqués sur la figure 3, lesquels polymorphismes mononucléotidiques sont le remplacement de l'adénine en position 218 par une thymine, le remplacement de l'adénine en position 224 par une thymine, et le remplacement de l'adénine en position 246 par une guanine.
